# EUROPÄISCHE PATENTSCHRIFT

(11) **EP 2 448 537 B1**
(45) Veröffentlichungstag und Bekanntmachung des Hinweises auf die Patenterteilung: **05.08.2015**
(21) Anmeldenummer: 10739497.5
(22) Anmeldetag: 28.06.2010
(51) Int. Cl.: A61F 13/20

(54) **PRESSE ZUR HERSTELLUNG EINES TAMPONS**
PRESS FOR PRODUCING A TAMPON
PRESSE POUR LA FABRICATION D'UN TAMPON

(30) Priorität: 29.06.2009 AT 10102009
(43) Veröffentlichungstag der Anmeldung: 09.05.2012
(73) Patentinhaber: Ruggli Projects AG, 6332 Hagendorn (CH)
(72) Erfinder: ROLLI, Kilian, CH-5400 Baden (CH); MÜLLER, Peter, CH-8004 Zürich (CH); HAMMEN, Axel, CH-5426 Lengnau (CH); GRABER, Heinz, CH-8962 Bergdietikon (CH); MADL, Viktor, CH-5425 Schneisingen (CH)
(74) Vertreter: Burger, Hannes
(86) Internationale Anmeldenummer: PCT/EP2010/003841
(87) Internationale Veröffentlichungsnummer: WO 2011/000507

(56) Entgegenhaltungen:
- WO-A2-2008/095937
- DE-A1-102005 050 514

## Beschreibung

Die Erfindung betrifft eine Presse zur Herstellung eines Tampons durch radiales Pressen eines absorbierenden Materials mittels Pressbacken, welche je ein Eindringungssegment zum Einprägen von Rillen in das absorbierende Material aufweisen.

Weiters betrifft die Erfindung ein Verfahren zur Herstellung eines Tampons durch radiales Pressen eines absorbierenden Materials mittels einer Presse mit Pressbacken, wobei die Pressbacken je ein Eindringungssegment zum Einprägen von Rillen in das absorbierende Material aufweisen.

Tampons sind an sich bekannte Hygieneartikel und können aus Wattebändern durch Rollen und anschließendem Verdichten in einer Pressvorrichtung hergestellt werden, wobei sich üblicherweise eine herstellungsbedingt kreiszylinderförmige Grundform des Tampons ergibt. Aus dem Stand der Technik sind seit langem viele unterschiedliche Tampons bekannt, deren Oberflächeneigenschaften physikalisch und/oder chemisch verändert werden, um sowohl ästhetische als auch funktionelle Vorteile zu verleihen. In diesem Zusammenhang sind Pressvorrichtungen bekannt geworden, um Tampons mit uneinheitlicher Oberflächentopografievor allem in Form von Längsrillen, gegebenenfalls unterschiedlicher Tiefe, in der Oberfläche des Tampons - herzustellen. Eine derartige Presse der eingangs genannten Art ist beispielsweise aus der EP 0639 363 oder der WO-2008/095937 bekannt geworden. Die bekannte Presse weist radial zu einem Tampon verschiebbare Pressbacken auf, welche je ein Eindringungssegment zum Eindringen in das absorbierende Material und eine Druckschulter umfassen. Die Eindringungssegmente sind hierbei als in den Pressbereich ragende Leisten mit geraden Seitenflächen ausgebildet, wobei die Längserstreckung der Leisten im wesentlichen parallel zur Längsachse des zu bearbeitenden Tampons verläuft.

Nachteilig an dieser Ausführungsform ist vor allem, dass sich in Bezug auf die Oberfläche des Tampons nur geradlinig verlaufende Rillen herstellen lassen. Rillen welche nach außen hin sichtbar eine Wellenform auf der Oberfläche des Tampons bilden, lassen sich jedoch mit dieser Presse nicht herstellen. Mit der Herstellung gewellter Rillen ist darüber hinaus das Problem verbunden, dass ein Ersetzen aller geraden Eindringungssegmente durch wellenförmige Eindringungssegmente dazu führen würde, dass ein Ausstoßen des Tampons aus der Presse erheblich erschwert werden würde.

Es ist daher eine Aufgabe der Erfindung, die oben genannten Nachteile des Stands der Technik zu überwinden und die Herstellung von Tampons mit an der Oberfläche des fertigen Tampons als gewellte Linien sichtbare gewellte Rillen herzustellen.

Diese Aufgabe wird mit einer Presse der eingangs genannten Art erfindungsgemäß dadurch gelöst, dass alternierend ein Pressbacken dessen Eindringungssegment als in den Pressraum ragende Leiste mit ebenen Seitenflächen und ein Pressbacken dessen Eindringungssegment als in den Pressraum ragender Steg mit gewellten Seitenflächen ausgebildet ist, angeordnet sind, wobei eine Projektion der Leiste auf eine Mantelfläche eines sich im Pressraum befindlichen Tampons eine gerade Linie und eine Projektion des gewellten Stegs auf die Mantelfläche eine gewellte Linie darstellt.

Die Erfindung ermöglicht es, wellenförmige Rillen in den Tampon einzuprägen, die als gewellte Linien auf dem fertigen Tampon sichtbar sind. Für einen Ausstoßvorgang können die Pressbacken mit den gewellten Eindringungssegmenten ganz oder teilweise von dem Tampon zurückgefahren werden. Die Pressbacken mit den geraden, in dem Tampon verbleibenden Leisten stellen sicher, dass der Tampon entlang dieser Eindringungssegmente bzw. von diesen geführt aus der Presse hinausgeschoben werden kann.

Gemäß einer vorteilhaften Variante der Erfindung kann die Leiste an einer Vorderkante mit Wellungen der Stege unmittelbar benachbarter Eindringungssegmente korrespondierende Aussparungen aufweisen. Dadurch lässt sich ein zuverlässiges Schließen der Presse gewährleisten, ohne dass es zu Klemmungen oder Stauchungen bzw. einer gegenseitigen Behinderung der geraden und der gewellten Eindringungselemente kommt.

Entsprechend der bevorzugten Variante der Erfindung weisen zwei Pressbacken mit gewellten Stegen, zwischen welchen ein Pressbacken mit einer geraden Leiste angeordnet ist, einen gegenläufigen Verlauf ihrer Wellungen auf.

Eine Ausführungsform der Erfindung, welche eine Bewegung des Tampons nach dem Pressen in Längsrichtung besonders gut ermöglicht, zeichnet sich dadurch aus, dass zwei unmittelbar aufeinander folgende Pressbacken mit geraden Leisten um einen Winkel von 90° zueinander versetzt sind, und zwei unmittelbar aufeinander folgende Pressbacken mit gewellten Stegen ebenfalls um 90° zueinander versetzt sind.

Gemäß der Erfindung verlaufen die Erzeugenden der gewellten Stege bevorzugt im wesentlichen normal zu einer Längsachse der Presse. Als Erzeugende eines gewellten Stegs werden hierbei die Mantellinien (also die kürzesten Verbindungsstrecken der Punkte des unteren Randes des Stegs mit den Punkten des oberen Randes des Stegs) verstanden, da sie den Mantel des Stegs "erzeugen".

Weiters können die Pressbacken in radialer Richtung jeweils geradlinig zur Längsmittelachse der Presse bewegbar sein.

Die oben genannte Aufgabe lässt sich auch mit einem Verfahren der eingangs genannten Art erfindungsgemäß dadurch lösen, dass alternierend ein Pressbacken dessen Eindringungssegment als in den Pressraum ragende Leiste mit ebenen Seitenflächen und ein Pressbacken, dessen Eindringungssegment als in den Pressraum ragender Steg mit gewellten Seitenflächen ausgebildet ist, angeordnet sind, wobei eine Projektion der Leiste auf eine Mantelfläche eines sich im Pressraum befindlichen Tampons eine gerade Linie und eine Projektion des gewellten Stegs auf die Mantelfläche eine gewellte Linie darstellt, wobei zum Einprägen der Rillen die Eindringungssegmente in den Tampon gepresst werden und nach einem Einprägen der Rillen in die Oberfläche des Tampons als gewellte Stege ausgeführte Eindringungssegmente aus dem absorbierenden Material des Tampons zurückgezogen werden während als Stege mit ebenen Seitenflächen ausgebildete Eindringungssegmente in dem absorbierenden Material als Führungen zum Ausstoßen des Tampons aus der Presse verbleiben.

Die Erfindung samt weiteren Vorteilen wird im Folgenden anhand einiger nicht einschränkender Ausführungsbeispiele näher erläutert, welche in den Zeichnungen dargestellt sind, In diesen zeigen:
- Fig. 1: eine Draufsicht auf eine erfindungsgemäße Presse mit auf einander in eine Pressstellung zugefahrenen Pressbacken;
- Fig. 2: zwei benachbarte Pressbacken der Presse aus Fig. 1 im näheren Detail;
- Fig. 3: eine Draufsicht auf die Presse aus Fig. 1 mit in eine geöffnete Stellung auseinander gefahrenen Pressbacken und eingelegtem Tampon;
- Fig. 4: die Presse aus Fig. 3 in einer Pressstellung;
- Fig. 5: die Presse aus Fig. 3 mit einem darin befindlichen Tampon nach einem Einprägen von Rillen mit zurückgefahrenen Eindringungselementen, welche wellenförmige Seitenflächen aufweisen;
- Fig. 6: eine Draufsicht auf eine erste Seite eines mit der Presse aus Fig. 1 hergestellten Tampons;
- Fig. 7: eine Draufsicht auf den Tampon aus Fig. 6 aus der Richtung y;
- Fig. 8: eine Ansicht der Tampons aus der Richtung x in Fig. 6;
- Fig. 9: einen Querschnitt entlang der Linie A-A in Fig. 6;
- Fig. 10: einen Querschnitt entlang der Linie B-B in Fig. 6;
- Fig. 11: einen Querschnitt entlang der Linie C-C in Fig. 5;
- Fig. 12: einen Querschnitt entlang der Linie D-D in Fig. 6 und
- Fig. 13: einen Querschnitt entlang der Linie E-E in Fig. 6.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

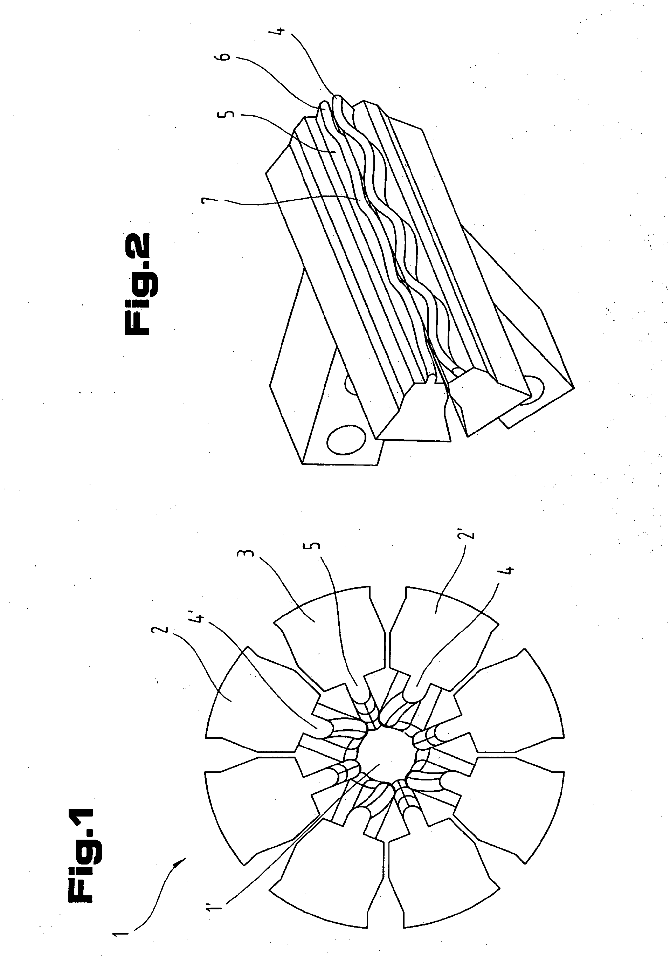

Gemäß Fig. 1 weist eine erfindungsgemäße Presse 1 Pressbacken 2, 3, welche in einer Sternformation in Bezug auf die Pressachse 1' angeordnet sind auf. Aufeinander folgende Pressbacken 2, 2', 3 sind somit je um einen vorgebbaren Winkel zueinander versetzt. Die Pressbacken können 2, 2', 3 hierbei in demselben radialen Abstand zu der Pressachse 1' angeordnet sein. Weiters können die Pressbacken 2, 2', 3 in Bezug auf die Pressachse 1' radial zwischen einer offenen und einer geschlossenen Position bewegt werden. Hierbei kann vorgesehen sein, dass die Pressbacken 2, 2', 3 auch einzeln oder in Gruppen radial verschoben werden können.

Die Pressung eines Tampons erfolgt durch eine Bewegung der Pressbacken 2, 2', 3 in Richtung der Pressachse 1', wobei die Längsachse des Tampons und die Pressachse 1' üblicherweise zusammenfallen zumindest aber im wesentlichen parallel zueinander verlaufen. Die Längsachse bezieht sich auf die längste lineare Abmessung des Tampons. Wie in Fig. 1 dargestellt kann die Presse acht Pressbacken 2, 2', 3 aufweisen. Die Anzahl der Pressbacken 2, 2', 3 ist jedoch nicht auf die dargestellte Anzahl beschränkt und kann je nach Gewicht und Zusammensetzung des Tampons variieren. Weiters können die Pressbacken 2, 2', 3 beheizt sein.

Die Pressbacken 2, 2', 3 weisen je ein Eindringungssegment 4, 4' und 5 zur Pressung des absorbierenden Materials in Form von Rillen auf. Weiters können die Pressbacken 2, 3 Druckschultern aufweisen, von welchen sich die Eindringungssegmente 4, 4' und 5 abheben. Die Eindringungselemente 4, 4' und 5 können sich jedoch auch direkt von den Pressbacken 2, 3 abheben.

Wie aus Fig. 2 ersichtlich, sind gemäß der Erfindung zwei Arten von Eindringungssegmenten 4, 4' und 5 bzw. zwei Arten von Pressbacken 2, 2', 3 vorgesehen. Eine erste Art von Eindringungssegmenten 5 ist je als Leiste mit ebenen Seitenflächen ausgebildet und eine zweite Art von Eindringungssegmenten 4 ist je als Steg mit gewellten Seitenflächen vorgesehen. Die Erzeugenden der gewellten Stege können hierbei im wesentlichen normal zur Pressachse 1' der Presse 1 verlaufen. Weiters verläuft die Längserstreckung der Eindringungssegmente 4, 4' und 5 im wesentlichen parallel zur Längsachse des Tampons 8.

In einem montierten Zustand der Pressbacken 2, 2', 3 ragen die Eindringungssegmente 4 und 5 in den Pressraum. Hierbei ergibt eine Projektion der Leiste auf eine Mantelfläche eines sich im Pressraum befindlichen Tampons eine gerade parallel zur Längserstreckung des Tampons verlaufende Linie und eine Projektion des gewellten Stegs auf die Mantelfläche eine gewellte Linie.

Gemäß der Erfindung sind alternierend ein Pressbacken 2, 2' mit einem Eindringungssegment 4, 4' welches, als gewellter Steg ausgebildet ist und ein Pressbacken 3 mit einem Eindringungssegment 5, welches als Leiste mit ebenen Seitenflächen geformt ist, angeordnet. Die gewellten Eindringungssegmente 4, 4' können in Bezug auf ein zwischen ihnen angeordnetes, gerades Eindringungssegment 5 jeweils spiegelsymmetrisch angeordnet sein. An einer Vorderkante 6 kann die Leiste mit Wellungen der Stege unmittelbar benachbarter Eindringungssegmente 4, 4' korrespondierende Aussparungen 7 aufweisen. Diese Aussparungen 7 befinden sich an den engsten Stellen zwischen den benachbarten gewellten Eindringungssegmenten 4, 4' und den geraden Eindringungssegmenten 5. Die soeben erwähnten engsten Stellen, d. h. Stellen, an welchen der Abstand zwischen den beiden seitlich des geraden Eindringungssegmentes 5 angeordneten gewellten Eindringungssegmente 4, 4' minimal wird, können als gemeinsame Knotenpunkte bzw. Knotenflächen der beiden gewellten Eindringungssegmente 4,4' betrachtet werden.

Aufgrund der Aussparungen 7 ergibt sich auch für die Vorderkante 6 der geraden Eindringungssegmente 5 eine mit den benachbarten, gewellten Stegen korrespondierende Form. Auf diese Weise wird verhindert, dass sich die beiden gewellten Stege und der gerade Steg während des Pressvorganges gegenseitig blockieren oder behindern.

Wie aus den Figuren 1, 3, 4 und 5 weiters ersichtlich ist, können zwei unmittelbar aufeinander folgende Pressbacken 3 mit geraden Leisten um einen Winkel von 90° zueinander versetzt sein, wobei zwei unmittelbar aufeinander folgende Pressbacken 2, 2' mit gewellten Stegen ebenfalls um 90° zueinander versetzt sein können. Die Erfindung ist jedoch nicht auf den angegebenen Winkel beschränkt, so können die Pressbacken 2, 2' und 3 im Prinzip um beliebige Winkel zueinander versetzt um den Pressraum angeordnet sein. Allerdings zeichnet sich die dargestellte Ausführungsform durch eine besonders gute Realisierbarkeit aus.

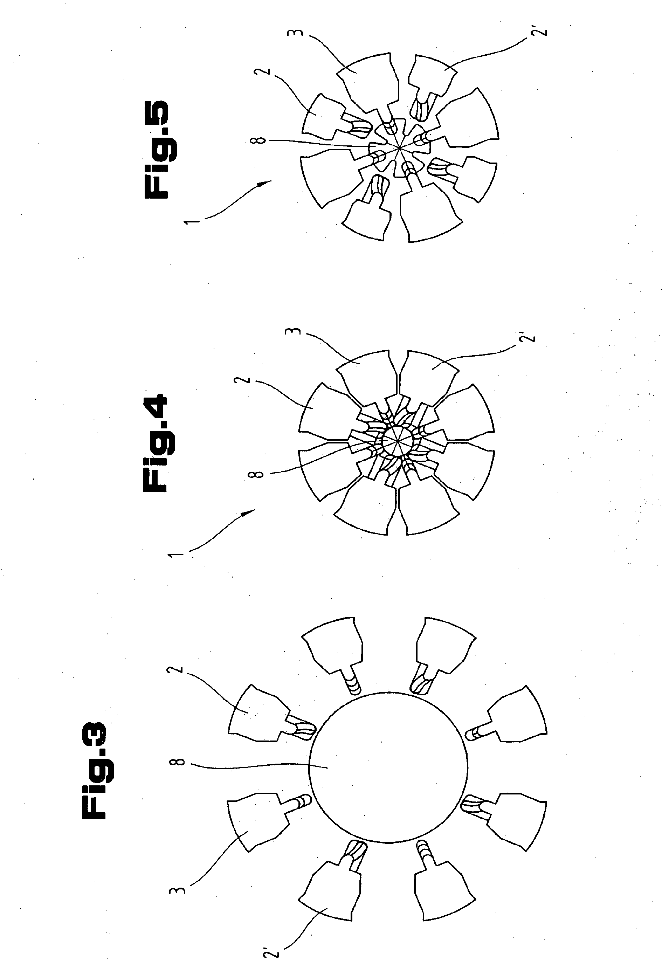

Gemäß Fig. 3 wird zur Ausformung der Rillen ein Tampon 8 in den zwischen den Pressbacken 2 und 3 liegende Pressbereich eingeführt. Hierauf werden die Pressbacken 2, 2' und 3 radial in Richtung des Tampons bewegt und dieses, wie in Fig. 4 dargestellt, gepresst und abschnittsweise verdichtet. Hierbei prägen die Eindringungssegmente 4, 4' und 5 in das Material des Tampons 8 Rillen ein. Infolge der gewellten Eindringungssegmente 4, 4' und der geraden Eindringungssegmente 5 ändert sich die Pressdichte der Tampons 8.

Fig. 5 zeigt die Pressvorrichtung mit einem darin eingespannten Tampon 8 in welchem bereits Rillenformen eingeprägt sind. Die Pressbacken 2, 2' mit den gewellten Eindringungssegmenten 4, 4' sind zurückgefahren und die geraden Eindringungssegmente 5 dienen als Führung für den Tampon 8 in einer parallel zu seiner Längsrichtung bzw. parallel zur Pressachse 1' verlaufenden Ausstoßrichtung. Der ausgestoßene Tampon 8 wird stromabwärts einer weiteren radialen Pressung seines Gesamtumfanges unterzogen.

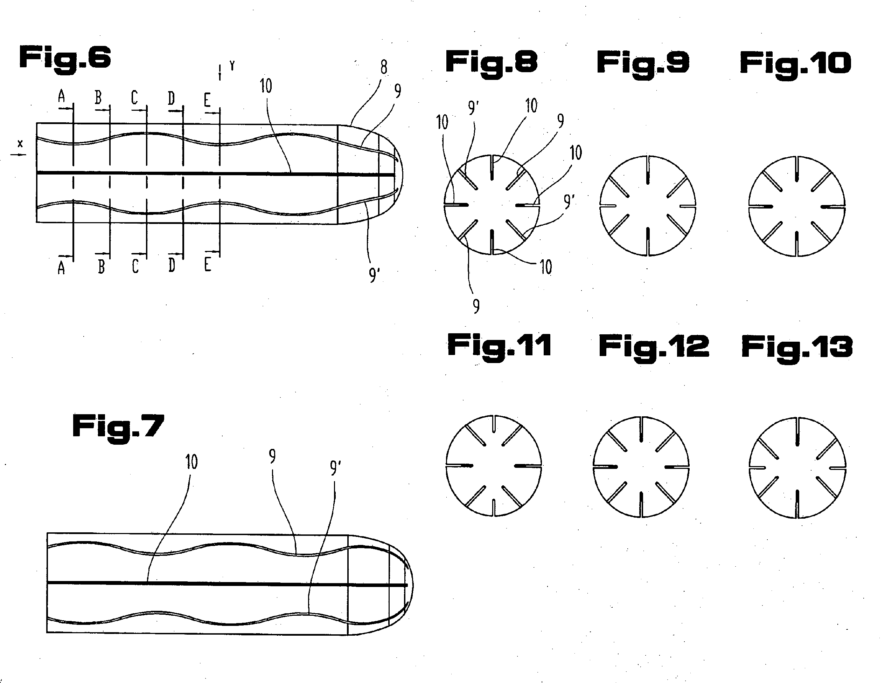

Gemäß Fig. 6 und 7 können mit der erfindungsgemäßen Presse einem Tampon Rillen 9, 9' in Form von Wellen, gepaart mit geraden Rillen 10 gegeben werden. Die benachbarte, gewellte Rillen 9, 9' des Tampons 8 können zueinander gegenläufig sein, wie aus den Darstellungen ersichtlich.

Figur 8 zeigt eine Ansicht aus Richtung x in Fig. 6. Die Figuren 9 bis 13 zeigen Querschnitte durch einen erfindungsgemäß hergestellten Tampon 8. Der Begriff Querschnitt bezieht sich hierbei auf eine Scheibe, die rechtwinklig zur Längsachse des Tampons 8 entnommen ist. Wie aus der sich ändernden Tiefe der resultierenden geraden Rillen ersichtlich ist, variiert die Eindringtiefe der geraden Eindringungssegmente 5 infolge der Aussparungen 7 an ihrer Stirnseite 6.

An dieser Stelle sei daraufhingewiesen, dass die Erfindung prinzipiell mit einer Kombination von geraden Eindringungssegmenten 5 und beliebigen Formen von anderen Eindringungssegmenten realisiert werden kann, die von geraden Rillen abweichende Rillenformen in die Oberfläche des Tampons 8 einprägen,

Die oben beschriebenen Ausführungsbeispiele beziehen sich auf mögliche Ausführungsvarianten einer erfindungsgemäßen Vorrichtung bzw. eines erfindungsgemäßen Verfahrens, wobei an dieser Stelle bemerkt sei, dass die Erfindung nicht auf die speziell dargestellten und beschriebenen Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvarianten möglich sind, vom Schutzumfang mit umfasst.

## Patentansprüche

1. Presse (1) zur Herstellung eines Tampons (8) durch radiales Pressen eines absorbierenden Materials mittels Pressbacken (2, 2', 3), welche je ein Eindringungssegment (4, . 5) zum Einprägen von Rillen (9, 9', 10) in das absorbierende Materials aufweisen, **dadurch gekennzeichnet, dass** alternierend ein Pressbacken (3) dessen Eindringungssegment (5) als in den Pressraum ragende Leiste mit ebenen Seitenflächen und ein Pressbacken (2, 2'), dessen Eindringungssegment (4, 4') als in den Pressraum ragender Steg mit gewellten Seitenflächen ausgebildet ist, angeordnet sind, wobei eine Projektion der Leiste auf eine Mantelfläche eines sich im Pressraum befindlichen Tampons (8) eine gerade Linie und eine Projektion des gewellten Stegs auf die Mantelfläche eine gewellte Linie darstellt.

2. Presse nach Anspruch 1, **dadurch gekennzeichnet, dass** die Leiste an einer Vorderkante (6) mit Wellungen der Stege unmittelbar benachbarter Eindringungssegmente (4, 4') korrespondierende Aussparungen (7) aufweist.

3. Presse nach Anspruch 1 oder 2, **dadurch gekennzeichnet, dass** zwei Pressbacken (2, 2') mit den gewellten Stegen, zwischen welchen ein Pressbacken (3) mit einer geraden Leiste angeordnet ist, einen gegenläufigen Verlauf ihrer Wellungen aufweisen.

4. Presse nach einem der Ansprüche 1 bis 3, **dadurch gekennzeichnet, dass** aufeinander folgende Pressbacken (2, 2',3) je um einen vorgebbaren Winkel zueinander versetzt sind.

5. Presse nach Anspruch 4, **dadurch gekennzeichnet, dass** zwei unmittelbar aufeinander folgende Pressbacken (3) mit geraden Leisten um einen Winkel von 90° zueinander versetzt sind, und zwei unmittelbar aufeinander folgende Pressbacken (2, 2') mit gewellten Stegen ebenfalls um 90° zueinander versetzt sind.

6. Presse nach einem der Ansprüche 1 bis 5, **dadurch gekennzeichnet, dass** die Erzeugenden der gewellten Stege im wesentlichen normal zu einer Pressachse (1') der Presse (1) verlaufen.

7. Presse nach einem der Ansprüche 1 bis 6, **dadurch gekennzeichnet, dass** die Pressbacken (2, 2', 3) in radialer Richtung jeweils geradlinig zur Pressachse (1') der Presse (1) bewegbar sind.

8. Verfahren zur Herstellung eines Tampons (8) durch radiales Pressen eines absorbierenden Materials mittels einer Presse (1) mit Pressbacken (2, 2', 3), wobei die Pressbacken (2, 2', 3) je ein Eindringungssegment (4, 5) zum Einprägen von Rillen (9, 9', 10) in das absorbierende Material aufweisen, **dadurch gekennzeichnet, dass** alternierend ein Pressbacken (3) dessen Eindringungssegment (5) als in den Pressraum ragende Leiste mit ebenen Seitenflächen und ein Pressbacken (2, 2') dessen Eindringungssegment (4, 4') als in den Pressraum ragender Steg mit gewellten Seitenflächen ausgebildet ist, angeordnet sind, wobei eine Projektion der Leiste auf eine Mantelfläche eines sich im Pressraum befindlichen Tampons (8) eine gerade Linie und eine Projektion des gewellten Stegs auf die Mantelfläche eine gewellte Linie darstellt, wobei zum Einprägen der Rillen (9, 10) die Eindringungssegmente (4, 5) in den Tampon (8) gepresst werden und nach einem Einprägen der Rillen (9, 10) in die Oberfläche des Tampons (8) als gewellte Stege ausgeführte Eindringungssegmente (4, 4') aus dem absorbierenden Material des Tampons (8) zurückgezogen werden während als Stege mit ebenen Seitenflächen ausgebildete Eindringungssegmente (5) in dem absorbierenden Material als Führungen zum Ausstoßen des Tampons (8) aus der Presse verbleiben.

## Claims

1. Press (1) for producing a tampon (8) by radially pressing an absorbent material by means of compression jaws (2, 2', 3) each of which has a penetrating segment (4, 5) for embossing grooves (9, 10) into the absorbent material, **characterized in that** one compression jaw (3) the penetrating segment (5) of which is designed as a strip having flat lateral faces projecting into the compression space and one compression jaw (2, 2') the penetrating segment (4, 4') of which is designed as a rib having undulated lateral faces projecting into the compression space are arranged so as to alternate, and a projection of the strip onto a peripheral surface of a tampon (8) present in the compression space is a straight line and a projection of the undulated rib onto the peripheral surface is an undulated line.

2. Press according to claim 1, **characterized in that** a strip at a leading edge (6) features recesses (7) corresponding to undulations of the ribs of directly adjacent penetrating segments (4, 4').

3. Press according to claim 1 or 2, **characterized in that** two compression jaws (2, 2') with undulated ribs, having a compression jaw (3) with a straight strip arranged between them, provide an opposed course of their undulations.

4. Press according to one of the claims 1 to 3, **characterized in that** compression jaws (2, 2', 3) directly following each other are each arranged at a pre-determinable angle.

5. Press according to claim 4, **characterized in that** two compression jaws (3) directly following one another and having straight strips are positioned at 90° relative to each other and two compression jaws (2, 2') directly following each other having undulated ribs are also positioned at 90° relative to each other.

6. Press according to one of the claims 1 to 5, **characterized in that** the generatrix of the undulated ribs essentially extend normal to a pressing axis (1') of the press (1).

7. Press according to one of the claims 1 to 6, **characterized in that** the each of the compression jaws (2, 2', 3) is moveable in radial direction and straight to the pressing axis (1') of the press each.

8. Method for producing a tampon (8) by radially pressing an absorbent material by means of a press (1) with compression jaws (2, 2', 3) and each of the compression jaws (2, 2', 3) has one penetrating segment (4, 5) for embossing grooves (9, 9', 10) into the absorbent material, **characterized in that** one compression jaw (3) the penetrating segment (5) of which is designed as a strip having flat lateral faces projecting into the compression space and one compression jaw (2, 2') the penetrating segment (4, 4') of which is designed as a rib having undulated lateral faces projecting into the compression space arranged so as to alternate, and a projection of the strip onto a peripheral surface of the tampon (8) present in the compression space is a straight line and a projection of the undulated rib onto the peripheral surface is an undulated line, and for embossing the grooves (9, 10) the penetrating segments (4, 5) are pressed into the tampon (8) and after having embossed the grooves (9, 10) into the surface of the tampon (8) penetrating segments (4, 4') embodied as undulated ribs are retracted from the absorbent material of the tampon (8) whereas penetrating segments (5) embodied as strips with flat lateral faces remain in the absorbent material as guidings for ejecting the tampon (8) out of the press.

## Revendications

1. Presse (1) pour la fabrication d'un tampon (8) par pression radiale sur un matériau absorbant à l'aide de mâchoires de presse (2, 2', 3) pourvues chacune d'un segment de pénétration (4, 5) pour la formation de rainures (9, 9', 10) dans le matériau absorbant, **caractérisée en ce que** sont disposées en alternance une mâchoire de presse (3) dont le segment de pénétration (5) présente la forme d'une baguette ayant des faces latérales planes et s'étendant jusque dans l'espace de presse et une mâchoire de presse (2, 2') dont le segment de pénétration (4, 4') présente la forme d'une barrette ayant des faces latérales ondulées et s'étendant jusque dans l'espace de presse, une projection de la baguette sur une surface enveloppante d'un tampon (8) situé dans l'espace de presse représentant une ligne droite et une projection de la barrette ondulée sur la surface enveloppante représentant une ligne ondulée.

2. Presse selon la revendication 1, **caractérisée en ce que** la baguette est pourvue, sur un bord avant (6), d'évidements (7) correspondant à des ondulations des barrettes de segments de pénétration (4, 4') immédiatement adjacents.

3. Presse selon la revendication 1 ou 2, **caractérisée en ce que** deux mâchoires de presse (2, 2') ayant des barrettes ondulées, entre lesquelles est disposée une mâchoire de presse (3) ayant une baguette droite, présentent des ondulations opposées.

4. Presse selon l'une des revendications 1 à 3, **caractérisée en ce que** des mâchoires successives (2, 2', 3) sont décalées chacune par rapport à l'autre d'un angle prédéterminé.

5. Presse selon la revendication 4, **caractérisée en ce que** deux mâchoires de presse (3) ayant des baguettes droites et se suivant directement sont décalées d'un angle de 90° l'une par rapport à l'autre, et **en ce que** deux mâchoires (2, 2') ayant des barrettes ondulées et se suivant directement sont également décalées l'une par rapport à l'autre d'un angle de 90°.

6. Presse selon l'une quelconque des revendications 1 à 5, **caractérisée en ce que** les génératrices des barrettes ondulées s'étendent sensiblement perpendiculairement à un axe de presse (1') de la presse (1).

7. Presse selon l'une quelconque des revendications 1 à 6, **caractérisée en ce que** les mâchoires de presse (2, 2', 3) sont mobiles chacune radialement en ligne droite par rapport à l'axe de presse (1') de la presse (1).

8. Procédé de fabrication d'un tampon (8) par pression radiale sur un matériau absorbant à l'aide d'une presse (1) ayant des mâchoires de presse (2, 2', 3), chacune des mâchoires de presse (2, 2', 3) étant pourvue d'un segment de pénétration (4, 5) pour la formation de rainures (9, 9', 10) dans le matériau absorbant, **caractérisée en ce que** sont disposées en alternance une mâchoire de presse (3) dont le segment de pénétration (5) présente la forme d'une baguette ayant des faces latérales planes et s'étendant jusque dans l'espace de presse et une mâchoire de presse (2, 2') dont le segment de pénétration (4, 4') présente la forme d'une barrette ayant des faces latérales ondulées et s'étendant jusque dans l'espace de presse, une projection de la baguette sur une surface enveloppante d'un tampon (8) situé dans l'espace de presse représentant une ligne droite et une projection de la barrette ondulée sur la surface enveloppante représentant une ligne ondulée, les segments de pénétration (4, 5) étant pressés dans le tampon (8) pour former les rainures (9, 10) et, après la formation des rainures (9, 10) dans la surface du tampon (8), des segments de pénétration (4, 4') ayant la forme de barrettes ondulées étant extraits du matériau absorbant du tampon (8) alors que des segments de pénétration (5) ayant la forme de baguettes avec des faces latérales planes restent dans le matériau absorbant comme guidages pour l'éjection du tampon (8) de la presse.
